# EUROPÄISCHE PATENTANMELDUNG

(11) **EP 1 985 289 A2**
(43) Veröffentlichungstag der Anmeldung: **29.10.2008**
(21) Anmeldenummer: 08162721.8
(22) Anmeldetag: 04.02.2003
(51) Int. Cl.: A61K 9/28

(54) **Schnelllöslicher Filmüberzug basierend auf Polyvinylalkohol-Polyether-Pfropfcopolymeren in Kombination mit Hydroxy-; Amid-; oder Esterfunktionen enthaltenden Komponenten**

(30) Priorität: 21.02.2002 DE 10207427
(62) Teilanmeldung aus: 03742450.4
(71) Anmelder: BASF SE, 67056 Ludwigshafen (DE)
(72) Erfinder: Kolter, Karl, 67117, Limburgerhof (DE); Angel, Maximilian, 67105, Schifferstadt (DE)

(57) **Zusammenfassung**

In Wasser schnelllösliches Filmüberzugsmittel zum Überziehen von pharmazeutischen, kosmetischen oder agrochemische Darreichungsformen, Saatgut, Nahrungsergänzungsmittel oder Lebensmitteln als festen Substraten, bestehend aus
a) 10 - 90 Gew.-% eines Polyvinylalkohol-Polyether-Pfropfcopolymeren (Komponente A),
b) 5-80 Gew.-% mindestens einer weiteren Komponente, die mindestens eine funktionelle Gruppe ausgewählt aus der Gruppe bestehend aus Hydroxy-, Amid- und Esterfunktionen enthält und ein Polymer ausgewählt aus der Gruppe bestehend aus Polysacchariden, Cellulosen, Stärken, Gelatine, Polyvinylpyrrolidonen, Vinylpyrrolidon-Vinylacetat-Copolymeren, Vinylpyrrolidon-Methacrylat-Copolymeren, Vinylpyrrolidon-Acrylat-Copolymeren, (Meth)-acrylat-Copolymeren, Hydroxyalkyl(meth)acrylat-Copolymeren, Polyvinylacetaten, Polylactiden, Polyethylenglykolen, Polypropylenglykolen oder Polyethylenglykol-Polypropylen-glykol-Blockcopolymeren und deren Derivaten, oder ein Zucker- oder Zuckeralkohol oder eine Derivat davon, Harnstoff oder eine hochdisperse Kieselsäure mit einer spezifischen Oberfläche ≥ 100 m²/g (Komponente B) darstellt, sowie
c) 0-70 Gew.-% weiteren üblichen Coatingbestandteilen (Komponenten C).

## Beschreibung

Die vorliegende Erfindung betrifft schnelllösliche Filmüberzüge zum Überziehen von festen Substraten wie pharmazeutischen, kosmetischen oder agrochemischen Darreichungsformen, Saatgut, Nahrungsergänzungsmitteln und Lebensmitteln, die aus mindestens einem Polyvinylalkohol-Polyether-Pfropfcopolymer (Komponente A), mindestens einer Komponente mit Hydroxy-, Amid-, oder Esterfunktionen (Komponente B) sowie optional weiteren üblichen Coatingbestandteilen (Komponenten C)bestehen. Weiterhin betrifft die Erfindung Verfahren zur Herstellung von trockenen Coatingprämixen und von wässrigen Coatinglösungen bzw. -suspensionen sowie deren Auftragen auf feste Darreichungsformen.

Feste Darreichungsformen werden aus den verschiedensten Gründen mit einem schnelllöslichen Überzug versehen. So kann beispielsweise das Aussehen, die Unterscheidbarkeit und die Schluckbarkeit verbessert werden, ein bitterer Geschmack überdeckt werden oder die Darreichungsform gegenüber äußeren Einflüssen wie z. B. Feuchtigkeit oder Sauerstoff geschützt werden. Da sich der Filmüberzug schnell in verschiedenen wässrigen Medien u. a. in künstlichem Magen- und Darmsaft lösen soll, muss der wichtigste Bestandteil der Coatingzubereitung ein wasserlösliches, filmbildendes Polymer sein. Zum Überziehen von Tabletten werden als filmbildende Polymere hauptsächlich Hydroxypropylmethylcellulose und Hydroxypropylcellulose eingesetzt, die jedoch gravierende Nachteile aufweisen. So ist die Viskosität dieser Polymere in Wasser sehr hoch und erlaubt nur eine Konzentration bis ca. 10 %, da aufgrund der hohen Viskosität bei höheren Konzentrationen keine feine Zerstäubung in der Sprühdüse mehr möglich ist und der Überzug rauh, inhomogen und unansehnlich wird. Weiterhin sind diese Polymere sehr spröde und erleiden häufig Risse während der Lagerung, insbesondere wenn der Kern durch Feuchtigkeitsaufnahme oder -abgabe sein Volumen verändert.

Polyvinylalkohol ist ebenfalls als Filmbildner bekannt, wird aber wegen verschiedener Nachteile selten eingesetzt. Die Verwendung von polyvinylalkoholhaltigen Zubereitungen, die aus Polyvinylalkohol, Weichmacher und Talkum bestehen, wird in WO 01/04195 beschrieben. Nachteilig bei diesen Zubereitungen sind die langsame Auflösung bei der Herstellung der wässrigen Coatinglösung, die hohe Viskosität, die niedrige Konzentration in der Sprühlösung, die Verwendung von Weichmachern und die langsame Auflösungsgeschwindigkeit des Filmüberzuges, insbesondere nach Lagerung sowie eine Versprödung des Filmüberzuges nach Lagerung einhergehend mit Rissbildungen.

Die Verwendung von Polyvinylalkohol-Polyether-Pfropfcopolymeren als Überzugsmittel oder Bindemittel in pharmazeutischen Darreichungsformen oder als Verpackungsmaterial oder als Zusatzstoff in kosmetischen, dermatologischen oder hygienischen Zubereitungen ist beispielsweise aus der WO 00/18375 bekannt. So wird beispielsweise eine Rezeptur für ein Filmüberzugsmittel beschrieben, welches aus einem Polyvinylalkohol-Polyether-Pfropfcopolymer und den üblichen Coatingbestandteilen zum Färben und Decken, nämlich Eisenoxid, Talkum und Titandioxid besteht. Ein so gearteter Überzug ist zwar flexibel, jedoch verhältnismäßig weich und zeigt, wenn Scherkräfte auf ihn einwirken, Abriebserscheinungen. Dies spielt besonders eine Rolle bei sehr großen Coatingansätzen, weil dann der hohe Druck bedingt durch die hohe Schüttung der Tabletten in Verbindung mit der rollierenden Bewegung der Tabletten in der Trommel entsprechend hohe Scherkräfte erzeugt. Da viele Arzneistoffe und auch einige Hilfsstoffe sehr lipophil sind, haften die Überzüge häufig schlecht auf der Tablettenoberfläche. Darüber hinaus sind Glätte und Glanz von solchen Coatingzubereitungen nicht zufriedenstellend.

Der Erfindung lag die Aufgabe zugrunde, einen Filmüberzug zu entwickeln, der sehr einfach und schnell in Wasser aufzulösen bzw. zu suspendieren ist, wodurch sich eine sehr kurze Herstellungszeit der Sprühzubereitung ergibt, der in hohen Polymer- und Feststoffkonzentrationen und mit hoher Sprührate zu versprühen ist, ohne dass die Sprühdüse verblockt, der sehr gut auf der Oberfläche spreitet, der flexibel ist und keinerlei Rissbildung während der Lagerung aufweist, der nicht klebrig ist, der auf allen Oberflächen gut haftet, der ausgezeichnete Glätte und Glanz aufweist, der gegenüber mechanischer Belastung sehr stabil ist und sich sehr schnell auflöst.

Die erfindungsgemäßen Filmüberzugsmittel bestehen aus
a) 10 - 90 Gew.-%, vorzugsweise 20 - 80 Gew.-% Polyvinylalkohol-Polyether-Pfropfcopolymeren (Komponenten A)
b) 5 - 80 Gew.-% vorzugsweise 10 - 70 Gew.-% mindestens einer weiteren Komponente, die ein Polymer ausgewählt aus der Gruppe bestehend aus Polysacchariden, Cellulosen, Stärken, Gelatine, Polyvinylpyrrolidonen, Vinylpyrrolidon-Vinylacetat-Copolymeren, Vinylpyrrolidon-Methacrylat-Copolymeren, Vinylpyrrolidon-Acrylat-Copolymeren, (Meth)-acrylat-Copolymeren, Hydroxyalkyl(meth)acrylat-Copolymeren, Polyvinylacetaten, Polylactiden, Polyethylenglykolen, Polypropylenglykolen oder Polyethylenglykol-Polypropylen-glykol-Blockcopolymeren und deren Derivaten, oder ein Zucker- oder Zuckeralkohol oder eine Derivat davon, Harnstoff oder eine hochdisperse Kieselsäure mit einer spezifischen Oberfläche ≥ 100 m²/g (Komponente B) darstellt,
c) sowie 0 - 70 % vorzugsweise 5 - 60 % weiteren üblichen Hilfsmitteln für Filmüberzüge.

Unter Polyvinylalkohol-Polyether-Pfropfcopolymeren werden Polymere verstanden, die erhältlich sind durch Polymerisation von
a) mindestens einem Vinylester von aliphatischen Ci-C₂₄-Carbonsäuren bevorzugt Vinylacetat, in Gegenwart von
b) Polyethern der allgemeinen Formel 1,

   R¹-O-(R²-O)ₓ-(R³-O)_{y}-(R⁴-O)_{z}-R⁵
c) in der die Variablen unabhängig voneinander folgende Bedeutung haben:
   - R¹: Wasserstoff, C₁-C₂₄-Alkyl, R⁶-C(=O)-, Polyalkoholrest; bevorzugt: R₁=H, CH₃-
   - R⁵: Wasserstoff, C₁-C₂₄-Alkyl, R⁶-C(=O)-; bevorzugt: R⁵=H
   - R²: bis R⁴
   -(CH₂)₂-, -(CH₂)₃₋, -(CH₂)₄₋, -CH₂-CH(CH₃)-,
   -CH₂-CH(CH₂-CH₃)-, -CH₂₋CHOR⁷-CH₂₋;
   bevorzugt R₂ bis R₄: ₋(CH₂)₂₋, -CH₂-CH(CH₃)- ganz besonders bevorzugt R₂ bis R₄: ₋(CH₂)₂₋
   - R₆: C₁-C₂₄-Alkyl;
   - R₇: Wasserstoff, C₁-C₂₄-Alkyl, R⁶-C(=O)-;
   - x: 1 bis 5000;
   bevorzugt: x = 10 bis 2000;
   ganz besonders bevorzugt: x = 20 bis 500
   - y: 0 bis 5000;
   bevorzugt: y = 0
   - z: 0 bis 5000;
   bevorzugt: z = 0,
mit der Massgabe, dass x ≥ 10 ist, wenn y und z = 0 sind, und anschließende vollständige oder teilweise Verseifung der Polyvinylestergruppen.
- x, y, z:: die Berechnung des Molekulargewichts des Polyethers aus x, y, z erfolgt als Mittelwert, da entsprechende Produkte meist eine breite Molgewichtsverteilung besitzen.

Bevorzugt sind Polyether mit einem mittleren Molgewicht zwischen 400 und 50000 g/mol, besonders bevorzugt 1500 bis 20000 g/mol.

Die Herstellung solcher Pfropfcopolymere ist an sich bekannt.

DE 1 077 430 beschreibt ein Verfahren zur Herstellung von Pfropfpolymerisaten von Vinylestern auf Polyalkylenglykole.

DE 1 094 457 und DE 1 081 229 beschreiben Verfahren zur Herstellung von Pfropfpolymerisaten von Polyvinylalkohol auf Polyalkylenglykolen durch Verseifung der Vinylester und deren Verwendung als Schutzkolloide, wasserlösliche Verpackungsfolien, als Schlichte- und Appreturmittel für Textilien und in der Kosmetik.

### Bevorzugt sind Polymere mit einem Verseifungsgrad der

Polyvinylestergruppen von > 70 mol %,
besonders bevorzugt > 80 mol % und
ganz besonders bevorzugt von > 85 mol %.

Besonders bevorzugt ist ein Polyvinylalkohol-Polyether-Pfropfcopolymer bei dem
a) Vinylacetat als zu pfropfendes Monomer verwendet wurde,
b) die Variablen folgende Bedeutung haben:
   - R¹: = H
   - R² - R⁴: = -(CH₂)₂₋
   - R₅: = H
   - x: = 20 bis 500
   - y: = 0
   - z: = 0
   und somit ein Polyethylenglykol mit einem mittleren Molekulargewicht von 6000 repräsentieren
c) der Verseifungsgrad der Estergruppen > 85 mol% liegt und
d) das Massenverhältnis der Molekülteile Polyvinylalkohol / Polyethylenglykol 6000 bei 75:25 liegt.

Weiterhin enthalten die Filmüberzugsmittel als Komponenten B Komponenten, die mindestens eine funktionelle Gruppe ausgewählt aus der Gruppe bestehend aus Hydroxy-, Amid- oder Esterfunktionen enthalten. Als Komponenten B können sowohl Polymere als auch niedermolekulare organische Verbindungen eingesetzt werden. Niedermolekular bedeutet erfindungsgemäß, dass es sich um eine organische Verbindung mit bis zu 20 C-Atomen handelt.

Diese Produkte sind in der Regel wasserlöslich, was im Sinne dieser Erfindung bedeutet, dass sich ≥ 1 g bei 25°C in 100 ml Wasser lösen. Wasserlöslich kann auch bedeuten, dass die Produkte sich pH-abhängig lösen. Bevorzugt lösen sich mehr als 5 g in 100 ml, besonders bevorzugt mehr als 20 g in 100 ml. Sie können aber auch wasserquellbar sein.

Als Polymere mit Hydroxy-, Amid- oder Esterfunktionen werden eingesetzt:
Polysaccharide, Cellulosen, Stärken, Polylactide, Polyethylenglykole, Polypropylenglykole oder Polyethylenglykol-Polypropylenglykol-Blockcopolymere einschließlich ihrer Derivate;
Polyvinylpyrrolidone, Vinylpyrrolidon-Vinylacetat-Copolymere;
Vinylpyrrolidon-Methacrylat-Copolymere, Vinylpyrrolidon-Acrylat-Copolymere;
(Meth)acrylat-Copolymere, Hydroxyalkyl- (meth)acrylat-Copolymere wie z. B. in DE 10049297 beschrieben, Polyvinylacetate,
Gelatine,
Bevorzugte Verbindungen sind:
   wasserlösliche Polymere wie, Vinylpyrrolidon-Vinylacetat 6:4-Copolymer (Copolyvidon), Polyvinylpyrrolidone mit einen K-Wert von 12 - 90, Hydroxypropylmethylcellulose, Hydroxypropylcellulose, Hydroxyethylcellulose, Hydroxyethylmethylcellulose, Methylcellulose, Natriumcarboxymethylcellulose, Amylose, Maltodextrine, Glucosesirupe, Cyclodextrine, Dextrane, Inulin, Polyfructose, Polydextrose, Alginate, Propylenglykolalginate, Pectine, Carrageenane, Guar, Tara, Xanthane, Gummi arabicum,
   pH-abhängig lösliche Polymere wie Butylmethacrylat-2-Dimethylaminoethylmethacrylat-Methylmethacrylat (1:2:1)-Copolymer, Methacrylsäure-Ethylacrylat (1:1)-Copolymer, Chitosane einschließlich deren wasserlöslicher Salze,
   wasserquellbare Polymere wie Ethylcellulose, quervernetztes Polyvinylpyrrolidon, Polyvinylacetat, Cellulose insbesondere mikrokristalline Cellulose.

Als niedermolekulare organische Verbindungen werden eingesetzt:

### Zucker, Zuckeralkohole oder deren Derivate;

Harnstoff

Bevorzugte Verbindungen sind:
Lactose, Saccharose, Glucose, Xylose, Mannit, Sorbit, Xylit, Isomalt (Palatinit®).
Weiterhin kann als Komponente B hochdisperse Kieselsäure mit einer spezifischen Oberfläche ≥ 100 m²/g eingesetzt werden.
Weiterhin können die Filmüberzüge als Komponenten C zusätzliche Hilfsstoffe enthalten, wie sie als Coatingbestandteile üblich sind. Weitere übliche Coatingbestandteile umfassen:

Farbgebende Komponenten:
Farbpigmente und Farbstoffe in wasserlöslicher oder wasserunlöslicher Form, z.B. Chinolingelblack, Tartrazinlack, Gelborangelack, FD&C Gelb-Aluminiumlack, Cochenillerotlack, Erythrosinlack, Azorubinlack, Indigotinlack, Erythrosin, Brillantschwarz, Patentblau, Brilliantblau, Cochenillerot, Gelborange, Amaranth, FD&C Blau Nr. 1, Indigotin, Betacarotin
Weißpigmente zur Erhöhung der Deckkraft des Überzuges, z.B. Titandioxid, Talkum; Farbpigmente wie z.B. Eisenoxide
Antiklebemittel z.B. Talkum, Magnesiumstearat, Glycerolmonostearat
Füllstoffe wie z. B. Calciumhydrogenphosphate
Schaumhemmende bzw. zerstörende Stoffe wie z.B. Silicon, Simethicon, Octanol
Glanzverstärker, z.B. Wachse, Fettalkohol- oder Fettsäurederivate, Polyethylenglykole Tenside zur Verbesserung des Benetzungsverhaltens und der Spreitung, z.B. Natriumlaurylsulfat, Sorbitanfettsäureester oder ethoxilierte Sorbitanfettsäureester, ethoxilierte Ester von hydriertem Rizinusöl oder ethoxilierte Fettsäureester wie Polyoxyethylenglycerolricinolat-35 oder Polyoxyethylenglyceroltrihydroxystearat-40, Natriumdioctylsulfosuccinat.
pH regulierende Substanzen und Puffer wie z.B. Natriumcitrat, Citronensäure, Phosphatpuffer, Acetatpuffer
Weichmacher
Schutzkolloide
Durch die erfindungsgemäßen Kombinationen ergeben sich unvorhergesehene und überraschende Produkt- und Filmeigenschaften.

Werden beispielsweise die erfindungsgemäßen Polyvinylalkohol-Polyether-Pfropfcopolymeren mit Polyvinylalkoholen kombiniert, so zeigen sich synergistische Effekte dahingehend, dass bei bestimmten Verhältnissen die Reißdehnung, die die Flexibilität der Filme kennzeichnet, höher ist als die der einzelnen Komponenten. Dieses Phänomen tritt nicht nur bei mittleren und hohen Feuchten wie z. B. 54 % r. F. (relativer Feuchte)auf, sondern auch bei niedrigen Feuchten wie z. B. 11 % r. F. Unvorhersehbar war zudem, dass auch bei Lagerung die Flexibilität der erfindungsgemäßen Zubereitungen konstant ist. Von Polyvinylalkohol ist nämlich bekannt, dass er im Laufe der Zeit dramatisch versprödet und die Flexibilität nahezu gegen Null geht. Wahrscheinlich ordnen sich die Polyvinylalkoholmoleküle im Laufe der Lagerung in besonderer Weise an, so dass eine Art kristalliner Zustand entsteht, der wenig flexibel ist und leicht bricht. Durch die Kombination mit Polyvinylalkohol-Polyether-Pfropfcopolymeren wird diese Struktur gebrochen und die Filme behalten ihre Flexibilität auch bei Lagerung.

| Gew.-Teile | Reißdehnung bei 23°/ 54 % r. F. [%] | Klebrigkeit bei 30°/75 % r. F. |
|---|---|---|
| PVA-PEG-Pfropfcopolymer | 102 | 1,25 |
| PVA-PEG-Pfropfcopolymer Polyvinylalkohol | 169 | 1 |
| PVA-PEG-Pfropfcopolymer Polyvinylalkohol | 213 | 0,75 |
| PVA-PEG-Pfropfcopolymer Polyvinylalkohol | 195 | 0,50 |
| PVA-PEG-Pfropfcopolymer Polyvinylalkohol | 167 | 0,75 |
| Polyvinylalkohol | 160 | 1 |

- PVA-PEG-Pfropfcopolymer:: Polyvinylalkohol-Polyethylenglykol 6000 (75 : 25), Verseifungsgrad 94 mol% (die Angabe Polyethylenglykol 6000 bedeutet, dass als Polyether b) der Formel I ein Polyethylenglykol mit einem mittleren Molekulargewicht von 6000 verwendet wurde).
- Polyvinylalkohol:: Verseifungsgrad 88 mol%, Viskosität 4 mPas (einer 4%igen Lösung bei 20°C, DIN 53015).

| Gew.-Teile | Reißdehnung nach Herstellung | Reißdehnung nach Lagerung bei 23°C über 6 Monate | Reißdehnung nach Lagerung bei 23°C über 12 Monate |
|---|---|---|---|
| | [%] | [%] | [%] |
| PVA-PEG-Pfropf copolymer Polyvinylalkohol | 213 | 205 | 202 |
| Polyvinylalkohol | 160 | 30 | 5 |

- PVA-PEG-Pfropfcopolymer:: Polyvinylalkohol-Polyethylenglykol 6000 (75 : 25), Verseifungsgrad 94 mol%
- Polyvinylalkohol:: Verseifungsgrad 88 mol%, Viskosität 4mPas.

Ein ähnliches Bild mit einem synergistischen Effekt ergibt sich auch bei der Klebrigkeit, denn bestimmte Kombinationen zeigen niedrigere Klebrigkeiten als die einzelnen Komponenten. Die Klebrigkeit wurde nach Hoessel (Cometics and Toiletries 111 (8), 73ff (1996)) bestimmt, wobei ein Wert von 5 eine starke Klebrigkeit und ein Wert von 0 keine Klebrigkeit beschreibt. Je niedriger der Wert, desto geringer die Klebrigkeit.

Bei der Kombination der erfindungsgemäßen Polyvinylalkohol-Polyether-Pfropfcopolymeren mit Polyvinylpyrrolidon-Polyvinylacetat-Copolymeren treten ähnliche Besonderheiten zu Tage. Auch hier liegt die Dehnbarkeit der Kombinationen höher als die der einzelnen Komponenten bzw. höher als der Summe der anteiligen Werte. Dies ist umso überraschender, da reines Copolyvidon sehr spröde und nicht dehnbar ist.

| Gew.-Teile | | Reißdehnung bei 23°/ 54% r. F. [%] |
|---|---|---|
| PVA-PEG-Pfropfcopolymer | | 99 |
| PVA-PEG-Pfropfcopolymer | 7,5 | 114 |
| Copolyvidon | 2,5 | |
| PVA-PEG-Pfropfcopolymer | 6 | 119 |
| Copolyvidon | 4 | |
| PVA-PEG-Pfropfcopolymer | 5 | 137 |
| Copolyvidon | 5 | |
| PVA-PEG-Pfropfcopolymer | 3 | 40 |
| Copolyvidon | 7 | |
| Copolyvidon | | 0 |

- PVA-PEG-Pfropfcopolymer:: Polyvinylalkohol-Polyethylenglykol 6000 (75 : 25), Verseifungsgrad 94 mol%
- Copolyvidon:: Polyvinylpyrrolidon-Polyvinylacetat-(6:4)-Copolymer
Polyvinylalkohol-Polyether-Pfropfcopolymere sind in der Regel relativ weiche Polymere und für verschiedene Anwendungszwecke sind härtere Überzüge von Vorteil. Die erfindungsgemäßen Kombinationen führen zu erheblich erhöhten Zugfestigkeiten und E-Modulen, sind aber dennoch flexibel. Diese Wirkung entfalten nicht nur Polymere, sondern auch niedermolekulare Stoffe wie Zucker, Zuckeralkohole, Glucosesirupe oder Maltodextrine. Es ist darüber hinaus allgemein bekannt, dass Feststoffe, die in Filmüberzüge eingearbeitet werden, deren Festigkeit schwächen. Überraschenderweise zeigt sich bei Polyvinylalkohol-Polyether-Pfropfcopolymeren ein entgegengesetzter Effekt. Die Festigkeit steigt an wie den Beispielen mit mikrokristalliner Cellulose und hochdispersem Siliciumdioxid zu erkennen ist.

| Gew.-Teile | | Zugfestigkeit bei 23°C / 54% r. F. [N/mm2] |
|---|---|---|
| PVA-PEG-Pfropfcopolymer | | 9 |
| PVA-PEG-Pfropfcopolymer | 5 | 12 |
| Copolyvidon | 5 | |
| PVA-PEG-Pfropfcopolymer | 8 | 14 |
| Mannit | 2 | |
| PVA-PEG-Pfropfcopolymer | 8 | 17 |
| Mikrokristalline Cellulose | 2 | |
| PVA-PEG-Pfropfcopolymer | 8 | 12 |
| Maltodextrin DE 17 | 2 | |
| PVA-PEG-Pfropfcopolymer | 6 | 24 |
| Polyvinylalkohol | 4 | |
| PVA-PEG-Pfropfcopolymer | 8 | 19 |
| Carrageenan | 2 | |
| PVA-PEG-Pfropfcopolymer | 8 | 20 |
| Hochdisperses | | |
| Siliciumdioxid | 2 | |
| PVA-PEG-Pfropfcopolymer | 8 | 26 |
| Chitosan-HCI | 2 | |
| PVA-PEG-Pfropfcopolymer | 8 | 12 |
| Alginat | 2 | |
| PVA-PEG-Pfropfcopolymer | 8 | 13 |
| Poloxamer 188 | 2 | |
| PVA-PEG-Pfropfcopolymer | 8 | 14 |
| Gelatine 100 Bloom | 2 | |

- PVA-PEG-Pfropfcopolymer:: Polyvinylalkohol-Polyethylenglykol 6000 (75 : 25) Verseifungsgrad 94 mol%
- Zugfestigkeitsmessung:: an 100 mm dicken Polymerfilmen (aus wassriger Lösung gegossen)

Die beanspruchten Kombinationen führen überraschenderweise häufig zu einer reduzierten Viskosität der Sprühlösung bei gleichem Feststoffgehalt. Dadurch wird die Zerstäubung verbessert, die Gefahr von Düsenverblockungen und Anbackungen an der Sprühdüse reduziert, das Spreitungsverhalten der Sprühlösung auf der Tablettenoberfläche wird besser, der Filmüberzug gleichmäßiger, glatter und glänzender. Die Sprührate kann deutlich erhöht werden. Darüber hinaus lässt sich auch der Feststoffgehalt der Sprühzubereitung weiter steigern, wodurch der ganze Prozess schneller und kostengünstiger wird. Es können Sprühsuspensionen mit Feststoffgehalten bis zu 50 Gew.-% appliziert werden.

| Gew.-Teile | | Viskosität einer 20%igen Lösung [mPas] |
|---|---|---|
| PVA-PEG-Pfropfcopolymer | | 95 |
| PVA-PEG-Pfropfcopolymer | 5 | 60 |
| Copolyvidon | 5 | |
| PVA-PEG-Pfropfcopolymer | 8 | 65 |
| Mikrokristalline Cellulose | 2 | |
| PVA-PEG-Pfropfcopolymer | 8 | 61 |
| Maltodextrin DE 17 | 2 | |
| PVA-PEG-Pfropfcopolymer | 8 | 76 |
| Poloxamer 188 | 2 | |
| PVA-PEG-Pfropfcopolymer | 8 | 69 |
| Harnstoff | 2 | |
| PVA-PEG-Pfropfcopolymer | 8 | 69 |
| Isomalt | 2 | |
| PVA-PEG-Pfropfcopolymer | 8 | 58 |
| Lactose | 2 | |

- PVA-PEG-Pfropfcopolymer:: Polyvinylalkohol-Polyethylenglykol 6000 (75 : 25) Verseifungsgrad 94 mol%

Die erfindungsgemäßen Coatingzubereitungen haften besser auf den zu coatenden Darreichungsformen. Dies ermöglicht insbesondere das Coaten von sehr lipophilen Oberflächen, wie Tabletten, die höhere Anteile an lipophilen Wirkstoffen, Wachs oder Fetten enthalten. Übliche Coatingzubereitungen versagen hier, weil die Coatinglösung schlecht spreitet und schlecht haftet.

Die ausgezeichneten Benetzungseigenschaften zeigen sich auch in der hervorragenden Farbhomogenität des Überzuges. Es finden sich selbst bei dünnen Überzügen und hohen Feststoffkonzentrationen keine sogenannten Farbnester, die auf eine lokale hohe Farbstoffkonzentration zurückzuführen sind.

Die Filmüberzüge sind selbst bei sehr hohem Pigment- bzw. Feststoffanteil glatt und glänzend. Die Gravur ist wunderschön nachgebildet. Es kommt zu keinerlei Brückenbildungen oder Feststoffansammlungen in der Gravur. Die überzogenen Darreichungsformen besitzen ein ausgezeichnetes Erscheinungsbild.

Die Sauerstoffdurchlässigkeit der erfindungsgemäßen Zubereitungen ist niedrig, wodurch sauerstoffempfindliche Wirkstoffe im Kern besser geschützt werden können.

Durch verminderten oxidativen Abbau wird zudem die Stabilität der eingesetzten Farbstoffe erhöht.

Es sei an dieser Stelle nochmals betont, dass die erfindungsgemäßen Zubereitungen keinerlei Weichmacher bedürfen. Weichmacherfreiheit ist ein enormer Vorteil, weil Weichmacher oft zu Problemen bei der Lagerung von überzogenen Formen führen.

So kann der Weichmacher in den Kern migrieren und den Wirkstoff in seinen physikalischen und chemischen Eigenschaften verändern, der Film versprödet dadurch und neigt zur Rissbildung. Die meisten Weichmacher weisen zudem eine gewisse Flüchtigkeit auf, die ebenfalls zu einer Versprödung führt. Alle diese Nachteile sind bei den erfindungsgemäßen Coatings nicht vorhanden.

Die Herstellung der erfindungsgemäßen Coatingzubereitungen kann auf verschiedenen Wegen erfolgen.
1. Bei der Herstellung der Sprühlösung werden die einzelnen Komponenten nacheinander unter Rühren in Wasser eingetragen, wobei die Reihenfolge variiert werden kann. Üblicherweise erfolgt hierbei die Zugabe der unlöslichen Coatingbestandteile (Pigmente, Deckmittel) erst nach Auflösung der löslichen. Die erfindungsgemäßen Zubereitungen können aber auch direkt hintereinander, ohne die Auflösung der löslichen Bestandteile abzuwarten, zugesetzt werden. Der Nachteil hierbei ist, dass für jeden Sprühansatz viele einzelne Wiegeschritte erforderlich sind. Übliche Coatingzubereitungen erfordern einen Desagglomerierschritt bzw. Homogenisierschritt mittels eines Ultra-turrax, Hochdruckhomogenisierers oder einer Korundscheibenmühle. Bei den erfindungsgemäßen Coatingzubereitungen ist dieser zeitaufwendige Prozessschritt in der Regel nicht mehr notwendig. Es wird schon durch einfaches Rühren eine ausgezeichnete Homogenität und Dispersität der Zubereitung erzeugt. Die Polyvinylalkohol-Polyether-Pfropfcopolymere beschleunigen überraschenderweise die Auflösung der Hilfsstoffe mit Hydroxy-, Amid-, oder Esterstruktur und sorgen für eine schnelle und homogene Dispergierung der eingesetzten Feststoffe.
2. Aus den einzelnen Komponenten kann aber auch durch trockene Mischung ein sogenannter Prämix erzeugt werden, der beim Anwender nur einen Wiegeschritt erforderlich macht. Alternativ kann die Herstellung des Prämixes auch durch verschiedene Granulationsverfahren wie z. B. Trockengranulation, Kompaktierung, Feuchtgranulation, Wirbelschichtgranulation, Schmelzgranulation erfolgen. Diese Produkte sind ausgezeichnet redispergierbar, ebenfalls ohne Anwendung von stark scherenden Maschinen. Die Redispergierung erfolgt durch Einrühren der gesamten Zubereitung in Wasser mittels eines üblichen Rührers. Die Auflösungs- und Redispergierzeit ist noch kürzer als unter 1.
3. Aus einer wässrigen Lösung bzw. Dispersion von Polyvinylalkohol-Polyether-Pfropfcopolymer und den Hilfsstoffen mit Hydroxy-, Amid- oder Esterfunktion wird zunächst durch ein Trocknungsverfahren wie z. B. Sprühtrocknung, Walzentrocknung oder Wirbelschichttrocknung ein homogenes Pulver oder Granulat erzeugt. Dieses Pulver oder Granulat kann sowohl nach Methode 1 oder Methode 2 weiterverarbeitet werden. Besonders vorteilhaft ist hierbei die Herstellung eines Prämixes durch trockene Mischung mit den restlichen Coatingbestandteilen, da dieser Prämix nur eine extrem kurze Zeit zum Redispergieren benötigt.
4. Aus allen Einsatzstoffen außer dem Farbpigment, wird durch ein Trocknungsverfahren analog 3. ein Pulver oder Granulat (weißer Prämix) erzeugt, welches ausgezeichnet redispergierbar ist und keinerlei Entmischungstendenzen aufweist. Die Einarbeitung des Farbpigmentes kann durch trockenes Untermischen bzw. den unter 2. genannten Verfahren erfolgen. Das Farbpigment kann aber auch erst bei der Zubereitung der Sprühsuspension zusammen mit dem weißen Prämix eingerührt werden.
5. Aus allen Einsatzstoffen außer dem Farbpigment wird durch ein trockenes Mischverfahren oder ein Granulationsverfahren analog 2. ein Pulver oder Granulat (weißer Prämix) erzeugt, welches sehr gut redispergierbar ist und lagerstabil ist. Das Farbpigment kann erst bei der Zubereitung der Sprühsuspension zusammen mit dem weißen Prämix eingerührt werden, oder separat vor oder nach dem weissen Prämix. Dadurch kann der Anwender selbst verschiedene Farbtöne einstellen. Die ausgezeichneten Benetzungseigenschaften des weißen Prämixes sorgen für eine gleichmäßige Farbverteilung.

Die Zeit, die für die Herstellung der Sprühsuspension benötigt wird, ist nach Methode 2 kürzer als nach Methode 1 und nach den Methoden 3 und 4 in der Regel kürzer als nach Methode 2. Je inniger die Kombination von Polyvinylalkohol-Polyether-Pfropfcopolymer und den Hilfsstoffen mit Hydroxy-, Amid- oder Esterfunktion ist, desto schneller erfolgt die Auflösung dieser Produkte. Deshalb löst sich ein durch Sprühtrocknung hergestelltes Pulver aus Polyvinylalkohol-Polyether-Pfropfcopolymer und den Hilfsstoffen mit Hydroxy-, Amid- oder Esterfunktion schneller auf als die trockene Mischung. Bestes Beispiel hierfür ist die Kombination mit Polyvinylalkohol. Reiner Polyvinylalkohol benötigt in Wasser von Raumtemperatur mehrere Tage bis zur vollständigen Auflösung. Ein gemeinsam sprühgetrocknetes Produkt löst sich innerhalb von Minuten auf.

Die Prämixe der erfindungsgemäßen Kombinationen weisen generell den Vorteil auf, dass sie beim Zugeben und Einrühren in Wasser nicht klumpen und eine ausgezeichnete Pigmentverteilung und Homogenität aufweisen. Die Prämixe können verhältnismäßig schnell dem vorgelegten Wasser zugesetzt werden. Die Herstellung der Sprühsuspension kann somit schneller, einfacher und unter Verwendung von einfachen Rührwerkzeugen erfolgen. Schnelllaufende Rührer mit hoher Scherbeanspruchung, die zudem Luft in die Sprühsuspension einarbeiten und Schaum verursachen, sind nicht erforderlich. Gegebenenfalls kann auch mit festen oder flüssigen Entschäumern gearbeitet werden.

Die Herstellung von sogenannten weißen Prämixen, die alle Bestandteile bis auf den Farbstoff enthalten, ermöglichen dem Anwender bei der Herstellung der Sprühsuspension unterschiedliche Farbtöne durch Zugabe der jeweiligen Farbstoffe einzustellen. Ein weißer Prämix kann somit für alle Überzüge verwendet werden, wodurch sich enorme Kostenvorteile ergeben.

Es ist natürlich klar, dass der weiße Prämix ohne weitere Zusätze zur Herstellung von weißen Filmüberzügen verwendet werden kann.

Ferner können die erfindungsgemäßen Coatings auch für farblose, transparente Überzüge eingesetzt werden. Hierbei wird auf die Verwendung von wasserunlöslichen Bestandteilen insbesondere den Deckmitteln verzichtet. Solche transparenten Überzüge, die hauptsächlich aus dem Polyvinylalkohol-Polyether-Pfropfcopolymer und mindestens einem wasserlöslichen Hilfsstoff mit Hydroxy-, Amid-, oder Esterfunktion bestehen, sind aufgrund ihrer Flexibilität besonders für das Coating von verformbaren Darreichungsformen, wie Kapseln geeignet. Sie können aber auch als sogenanntes Topcoating auf eine vorhandene Coatingschicht aufgetragen werden und erhöhen so Glätte und Glanz.

Auflösungszeit zur Herstellung einer 20 gew.-%igen wässrigen Lösung bei Raumtemperatur mittels eines Flügelrührers bei 900 Umdrehungen/min.

| Gew.-Teile | | | Auflösungszeit |
|---|---|---|---|
| Polyvinylalkohol | | | 48 h * |
| Verseifungsgrad | 88 mol% | | |
| Viskosität | 4 mPas | | |
| PVA-PEG-Pfropfcopolymer | | | 10 min |
| PVA-PEG-Pfropfcopolymer | | 8 | 7 min |
| Lactose | | 2 | |
| Trockene Mischung | | | |
| PVA-PEG-Pfropfcopolymer | | 5 | 11 min |
| Polyvinylalkohol | | 5 | |
| Sprühgetrocknetes Produkt | | | |
| PVA-PEG-Pfropfcopolymer | | 5 | 6 min |
| Copolyvidon | | 5 | |
| Trockene Mischung | | | |
| PVA-PEG-Pfropfcopolymer | | 5 | 5 min |
| Copolyvidon | | 5 | |
| Sprühgetrocknetes Produkt | | | |
| PVA-PEG-Pfropfcopolymer | | 5 | 4 min |
| Mikrokristalline Cellulose | | 5 | |
| Trockene Mischung | | | |
| PVA-PEG-Pfropfcopolymer | | 5 | 7 min |
| Mannitol | | 5 | |
| Granulat | | | |

- nach 48 h sind immer noch ca. 3 % ungelöste Anteile vorhanden
- PVA-PEG-Pfropfcopolymer:: Polyvinylalkohol-Polyethylenglykol 6000 (75 : 25) Verseifungsgrad 94 mol%

Das Aufbringen des Filmüberzugs kann in allen für feste pharmazeutische, kosmetische, agrochemische Darreichungsformen, Saatgut, Nahrungsergänzungsmittel und Lebensmittel geeigneten Coatingeinrichtungen erfolgen wie z.B. Horizontaltrommelcoatern, Wirbelschichtcoatern, Tauchschwertcoatern, Dragierkesseln.

Für das Zerstäuben der Coatingzubereitung wird vorzugsweise eine Zweistoffdüse verwendet. Die Zulufttemperatur sollte zwischen 30 - 90°C vorzugsweise zwischen 40 - 80°C betragen.

Prinzipiell können alle Kernformen mit gewölbter, konvexer oder koncaver Oberfläche gecoatet werden, unabhängig davon, ob es sich um runde, vieleckige, oblong oder football-shape-Formen handelt.

Durch die niedrigen Viskositäten, die ausgezeichneten Benetzungs- und Spreitungseigenschaften wird eine einzigartige Auskleidung der Gravur erreicht. Es treten keine Brücken- oder Schmiereffekte in der Gravur auf.

Der Kern kann auch ein Subcoating tragen, welches in der Regel aufgebracht wird, um den Wirkstoff besonders zu schützen, z.B. vor Wasser, Sauerstoff, Protonen oder chemischen Stoffen des Überzuges sowie des Magen- und Darminhaltes.

Die erfindungsgemäßen Coatings können auch in mehreren Schichten aufgetragen werden, die sich in ihrer Zusammensetzung unterscheiden. So kann beispielsweise über ein gefärbtes Coating noch eine Schicht farbloses Coating appliziert werden.

Hinsichtlich der Wirkstoffe gibt es für die erfindungsgemäßen Darreichungsformen keine Beschränkungen. Es können Wirkstoffe aller Indikationsgebiete eingesetzt werden, Humanarzneistoffe wie Tierarzneistoffe, Vitamine, Carotinoide, Nutraceuticals, Nahrungsergänzungsstoffe, Mineralstoffe, Mikronährstoffe etc. Die Wirkstoffe können unterschiedliche physikochemische Eigenschaften wie Lipophilie, Löslichkeit, Korngröße, Kornstruktur, Oberfläche etc. besitzen.

Die zu coatenden Darreichungsformen können als Tabletten, Kapseln, Extrudate, Pellets, Granulate, Kristalle, Pulver, Saatgut oder Lebensmittelformen vorliegen.

Die Kombination von Polyvinylalkohol-Polyether-Pfropfcopolymeren mit Hilfsstoffen, die eine oder mehrere Hydroxy-, Amid- oder Esterfunktionen tragen, führt überraschenderweise zu erheblich verbesserten Coatingeigenschaften.

Die erfindungsgemäßen Filmüberzugsmischungen lassen sich sehr einfach und schnell in Wasser lösen bzw. dispergieren, besitzen niedrige Viskositäten, enorm hohe Flexibilitäten, gute Festigkeiten, niedrige Klebrigkeiten und lassen sich in hoher Feststoffkonzentration mit hoher Sprührate auf feste Darreichungsformen aufbringen. Die überzogenen Darreichungsformen sind sehr glatt, glänzend, gleichmäßig gefärbt, sehr schnell zerfallend und lagerstabil.

### Beispiele

Die Prozentangaben beziehen sich, soweit nichts anderes angegeben ist, auf Gew.-%.

### Beispiel 1

| Coatingzusammensetzung: | |
|---|---|
| Polyvinylalkohol-Polyethylenglykol 6000 (75:25) | 35% |
| Pfropfcopolymer (Verseifungsgrad 94 mol%) | |
| Vinylpyrrolidon-Vinylacetat 6:4 Copolymer | 35% |
| (Copolyvidon) | |
| Talkum | 20% |
| Titandioxid | 5% |
| Eisenoxid rot | 5% |

### Herstellung:

Alle Bestandteile wurden in der oben aufgeführten Reihenfolge direkt nacheinander mittels eines Flügelrührers in Wasser eingerührt, so dass eine Sprühzubereitung mit einem Feststoffgehalt von 30% entstand. Die Auflösung bzw. Dispergierung war nach 17 min abgeschlossen. Die Sprühsuspension war niedrig viskos und homogen.

Die Sprühsuspension wurde in einen Horizontaltrommelcoater (24" Accela-Cota) auf 5 kg Propranolol-Tabletten folgender Zusammensetzung aufgesprüht:

| | |
|---|---|
| Propranolol-HCI | 40 mg |
| Ludipress^{®} (BASF AG) ¹⁾ | 97,5 mg |
| Copolyvidon | 12,5 mg |
| Mikrokristalline Cellulose ²⁾ | 97,5 mg |
| Magnesiumstearat | 2,5 mg |
| Gesamtgewicht | 250 mg |

| | |
|---|---|
| Durchmesser: 9 mm, gewölbt ¹⁾ Formuliertes Produkt aus 93 Gew.-% Lactose, 3,5 Gew.-% Povidon und 3,5 Gew.-% Crospovidon ²⁾ mittlere Teilchengröße 100 mm | |

| Sprühbedingungen: | |
|---|---|
| Zulufttemperatur | 70°C |
| Ablufttemperatur | 38°C |
| Sprührate | 50 g/min |
| Sprühdruck | 4 bar |
| Auftragsmenge | 634 g Sprühdispersion entspr. |
| | 190 g Feststoff |
| Sprühzeit | 13 min |

| Filmtabletteneigenschaften: | |
|---|---|
| Bruchfestigkeit | 120 N |
| Friabilität | 0 % |
| Zerfallszeit | 5:25 (min:s) |
| Freisetzung | 20 min: 100% |

Der Überzug war glatt, gleichmäßig und homogen. Die Gravur war schön nachgebildet, ohne Schmiereffekte oder Brückenbildungen. Es war keine Verlängerung der Zerfallszeit oder der Wirkstofffreisetzung gegenüber dem Kern festzustellen. Die Bruchfestigkeit war gegenüber dem Kern um 27N höher. Während der Stabilitätsprüfung über 12 Monate wurden keine Veränderungen an den Eigenschaften der Filmtabletten festgestellt.

### Beispiel 2

| Coatingzusammensetzung: | |
|---|---|
| Polyvinylalkohol-Polyethylenglykol 6000 (75:25) | 40 % |
| Pfropfcopolymer (Verseifungsgrad 94 mol%) | |
| Vinylpyrrolidon-Vinylacetat 6:4 Copolymer (Copolyvidon) | 30 % |
| Talkum | 20 % |
| Titandioxid | 5 % |
| Eisenoxid gelb | 5 % |

### Herstellung:

Alle Bestandteile wurden in einem Turbulamischer 10 min gemischt. Diese Prämixmischung wurde mittels eines Flügelrührers in Wasser eingerührt, so dass eine Sprühzubereitung mit einem Feststoffgehalt von 35 % entstand. Die Auflösung bzw. Dispergierung war nach 13 min abgeschlossen. Die Sprühsuspension war niedrig viskos und homogen.

Die Sprühsuspension wurde in einen Horizontaltrommelcoater (24" Accela-Cota) auf 5 kg Propranolol-Tabletten aufgesprüht:

| Sprühbedingungen: | |
|---|---|
| Zulufttemperatur | 71°C |
| Ablufttemperatur | 40°C |
| Sprührate | 55 g/min |
| Sprühdruck | 4 bar |
| Auftragsmenge | 543 g Sprühdispersion entspr. 190 g Feststoff |
| Sprühzeit | 10 min |

| Filmtabletteneigenschaften: | |
|---|---|
| Bruchfestigkeit | 118 N |
| Friabilität | 0 % |
| Zerfallszeit | 5:15 (min:s) |
| Freisetzung | 20 min: 100% |

Der Überzug war glatt, gleichmäßig und homogen. Die Gravur war schön nachgebildet, ohne Schmiereffekte oder Brückenbildungen. Es war keine Verlängerung der Zerfallszeit oder der Wirkstofffreisetzung gegenüber dem Kern festzustellen. Die Bruchfestigkeit war gegenüber dem Kern um 25N höher. Während der Stabilitätsprüfung über 12 Monate wurden keine Veränderungen an den Eigenschaften der Filmtabletten festgestellt.

### Beispiel 3

| Coatingzusammensetzung: | |
|---|---|
| Polyvinylalkohol-Polyethylenglykol 6000 (75:25) | 40 % |
| Pfropfcopolymer (Verseifungsgrad 94 mol%) | |
| Polyvinylalkohol (Verseifungsgrad 88 mol%, Viskosität 4mPas) | 27 % |
| Talkum | 24 % |
| Titandioxid | 7 % |
| Indigotinlack | 2 % |

### Herstellung:

Eine Lösung von Polyvinylalkohol-Polyether-Pfropfcopolymer und Polyvinylalkohol mit einem Feststoffgehalt von 30% wurde sprühgetrocknet. Dieses Pulver wurde in einem Turbulamischer mit Talkum, Titandioxid und Indigotinlack 10 min zu einem Prämix gemischt.

Zur Herstellung der Sprühsuspension wurde der Prämix mittels eines Flügelrührers in Wasser eingerührt, so dass eine Sprühzubereitung mit einem Feststoffgehalt von 30% entstand. Die Auflösung bzw. Dispergierung war nach 15 min abgeschlossen. Die Sprühsuspension war niedrig viskos und homogen.

Die Sprühsuspension wurde in einen Horizontaltrommelcoater (24" Accela-Cota) auf 5 kg Coffein-Tabletten folgender Zusammensetzung aufgesprüht:

| | |
|---|---|
| Coffein | 50 mg |
| Ludipress^{®} (BASF AG) | 229 mg |
| Mikrokristalline Cellulose ¹⁾ | 40 mg |
| Crospovidon | 10 mg |
| Magnesiumstearat | 1 mg |
| Gesamtgewicht | 330 mg |

| Sprühbedingungen: | |
|---|---|
| Zulufttemperatur | 70°C |
| Ablufttemperatur | 41 °C |
| Sprührate | 48 g/min |
| Sprühdruck | 4,5 bar |
| Auftragsmenge | 520 g Sprühdispersion entspr. 150 g Feststoff |
| Sprühzeit | 11 min |

| Filmtabletteneigenschaften: | |
|---|---|
| Bruchfestigkeit | 131 N |
| Friabilität | 0 % |
| Zerfallszeit | 0:48 (min:s) |
| Freisetzung | 20 min: 100% |

| | |
|---|---|
| Durchmesser: 9 mm, gewölbt ¹⁾ mittlere Teilchengröße 100 mm | |

Der Überzug war glatt, gleichmäßig und homogen. Die Gravur war schön nachgebildet, ohne Schmiereffekte oder Brückenbildungen. Es war keine Verlängerung der Zerfallszeit oder der Wirkstofffreisetzung gegenüber dem Kern festzustellen. Die Bruchfestigkeit war gegenüber dem Kern um 24N höher. Während der Stabilitätsprüfung über 12 Monate wurden keine Veränderungen an den Eigenschaften der Filmtabletten festgestellt.

### Beispiel 4

| Coatingzusammensetzung | |
|---|---|
| Polyvinylalkohol-Polyethylenglykol 6000 (75:25) | 60 % |
| Pfropfcopolymer (Verseifungsgrad 94 mol%) | |
| Polyvinylalkohol (Verseifungsgrad 88 mol%, Viskosität 4 mPas) | 40 % |

### Herstellung

Das sprühgetrocknete Pulver aus Polyvinylalkohol-Polyether-Pfropfcopolymer und Polyvinylalkohol aus Beispiel 3 wurde mittels eines Flügelrührers in Wasser gelöst, so dass eine Feststoffkonzentration von 24 % entstand. Die Auflösung war nach 15 min abgeschlossen. Diese Lösung wurde in einem Horizontaltrommelcoater (24" Accela-Cota) bei einer Zulufttemperatur von 60°C auf Weichgelatinekapseln gesprüht.

Die Weichgelatinekapseln wiesen einen glatten, gleichmäßigen, extrem flexiblen, schnell auflösenden Überzug auf, der auch mechanischen Belastungen wie Druck und Zug standhielt. Während der Lagerung über 12 Monate ergaben sich keine Veränderungen.

### Beispiel 5

| Coatingzusammensetzung: | |
|---|---|
| Polyvinylalkohol-Methylpolyethylenglykol 1500 (80:20) | 55 % |
| Pfropfcopolymer (Verseifungsgrad 96 mol%) | |
| Mannit | 20 % |
| Talkum | 15 % |
| Titandioxid | 6 % |
| Eisenoxid rot | 3,5 % |
| Polydimethylsiloxan (Dimeticon) | 0,5 % |

### Herstellung:

Alle Bestandteile wurden in einem Glatt-Wirbelschichtgranulator bei einer Zulufttemperatur von 70°C durch Einsprühen von 30% (bezogen auf die Gesamtmischung) Wasser granuliert.

Aus diesem Prämixgranulat wurde durch Einrühren in Wasser mittels eines Flügelrührers eine Sprühsuspension mit einem Feststoffgehalt von 30 % hergestellt. Schon nach 10 min war die Zubereitung vollständig redispergiert und gebrauchsfertig. Die Applikation auf die Tabletten erfolgte analog Beispiel 1.

### Beispiel 6

| Coatingzusammensetzung: | |
|---|---|
| Polyvinylalkohol-Polypropylenglykol-(70:30)-Pfropfcopolymer | 55 % |
| (Verseifungsgrad 97 mol%) | |
| Lactose | 28,5 % |
| Talkum | 10 % |
| Titandioxid | 5 % |
| Chinolingelblack | 1,5 % |

### Herstellung:

Alle Bestandteile wurden in einem Stephanmischer mit 15 % (bezogen auf die Gesamtmischung) Wasser besprüht, durch ein Sieb mit einer Maschenweite von 1,0 mm gegeben und abgetrocknet.

Aus diesem Prämixgranulat wurde durch Einrühren in Wasser mittels eines Flügelrührers eine Sprühsuspension mit einem Feststoffgehalt von 35 % hergestellt. Schon nach 10 min war die Zubereitung vollständig redispergiert und gebrauchsfertig. Die Applikation auf die Tabletten erfolgte analog Beispiel 2.

### Beispiel 7

| Coatingzusammensetzung: | |
|---|---|
| Polyvinylalkohol-Polyethylenglykol 4000 (75:25)-Pfropfcopolymer | 55 % |
| (Verseifungsgrad 96 mol%) | |
| Mikrokristalline Cellulose | 30 % |
| mittlere Teilchengröße 20 mm | |
| Talkum | 6 % |
| Titandioxid | 5 % |
| Eisenoxid rot | 3,7 % |
| Natriumlaurylsulfat | 0,3 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 40 % hergestellt. Schon nach 16 min war die Zubereitung vollständig redispergiert und gebrauchsfertig. Die Applikation auf die Tabletten erfolgte analog Beispiel 3.

### Beispiel 8

| Coatingzusammensetzung: | |
|---|---|
| Polyvinylalkohol-Polyethylenglykol 6000 (75:25)-Pfropfcopolymer | 71 % |
| (Verseifungsgrad 96 mol%) | |
| Hochdisperses Siliciumdioxid (Aerosil 200) | 10 % |
| Talkum | 10 % |
| Titandioxid | 6 % |
| Chinolingelblack | 3 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 25 % hergestellt. Schon nach 10 min war die Zubereitung vollständig redispergiert und gebrauchsfertig. Die Applikation auf die Tabletten erfolgte analog Beispiel 3.

### Beispiel 9

| Coatingzusammensetzung: | |
|---|---|
| Polyvinylalkohol-Polyethylenglykol 6000 (85:15)-Pfropfcopolymer | 60 % |
| (Verseifungsgrad 92 mol%) | |
| Maltodextrin DE Wert 17 | 20 % |
| Titandioxid | 12 % |
| Eisenoxid rot | 7,5 % |
| Natriumdioctylsulfosuccinat | 0,5 % |

### Herstellung:

Alle Bestandteile wurden in einem Turbulamischer 10 min gemischt und auf einem Walzenkompaktor (Bepex) kompaktiert und durch ein Sieb von 1,5 mm Maschenweite gedrückt.

Aus diesem Prämix wurde durch Einrühren in Wasser mittels eines Flügelrührers eine Sprühsuspension mit einem Feststoffgehalt von 25 % hergestellt. Schon nach 11 min war die Zubereitung vollständig redispergiert und gebrauchsfertig. Die Applikation auf die Tabletten erfolgte analog Beispiel 1.

### Beispiel 10

| Coatingzusammensetzung: | |
|---|---|
| Polyvinylalkohol-Polyethylenglykol 6000 (75:25)-Pfropfcopolymer (Verseifungsgrad 94 mol%) | 62 % |
| Chitosan Hydrochlorid | 8 % |
| Talkum | 15 % |
| Titandioxid | 13 % |
| Betacarotin-Trockenpulver | 2 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 25 % hergestellt. Schon nach 13 min war die Zubereitung vollständig redispergiert und gebrauchsfertig. Die Applikation auf die Tabletten erfolgte analog Beispiel 1.

### Beispiel 11

| Coatingzusammensetzung: | |
|---|---|
| Polyvinylalkohol-Polyethylenglykol 1500 (75:25)-Pfropfcopolymer (Verseifungsgrad 96 mol%) | 62 % |
| Harnstoff | 15 % |
| Calciumhydrogenphosphat | 15 % |
| Titandioxid | 5 % |
| Gelborangelack | 3 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 27 % hergestellt. Schon nach 12 min war die Zubereitung vollständig redispergiert und gebrauchsfertig. Die Applikation auf die Tabletten erfolgte analog Beispiel 1.

### Beispiel 12

| Coatingzusammensetzung: | |
|---|---|
| Polyvinylalkohol-Polyethylenglykol 6000 (75:25)-Pfropfcopolymer | 62 % |
| (Verseifungsgrad 94 mol%) | |
| Isomalt | 15 % |
| Calciumhydrogenphosphat | 15 % |
| Talkum | 10 % |
| Titandioxid | 5 % |
| Patentblau | 3 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 25 % hergestellt. Schon nach 11 min war die Zubereitung vollständig redispergiert und gebrauchsfertig. Die Applikation auf die Tabletten erfolgte analog Beispiel 1.

### Beispiel 13

| Coatingzusammensetzung: | |
|---|---|
| Polyvinylalkohol-Polyethylenglykol 6000 (75:25)-Pfropfcopolymer | 62 % |
| (Verseifungsgrad 94 mol%) | |
| Carrageenan | 15 % |
| Talkum | 15 % |
| Titandioxid | 5 % |
| Chinolingelblack | 3 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 24 % hergestellt. Schon nach 15 min war die Zubereitung vollständig redispergiert und gebrauchsfertig. Die Applikation auf die Tabletten erfolgte analog Beispiel 3.

### Beispiel 14

| Coatingzusammensetzung: | |
|---|---|
| Polyvinylalkohol-Polyethylenglykol 4000 (80:20)-Pfropfcopolymer | 55 % |
| (Verseifungsgrad 96 mol%) | |
| Polyethylenoxid-Polypropylenoxid-Blockcopolymer | 12 % |
| Poloxamer 188 | |
| Talkum | 20 % |
| Titandioxid | 10 % |
| Azorubinlack | 3 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 28 Gew.-% hergestellt. Schon nach 13 min war die Zubereitung vollständig redispergiert und gebrauchsfertig. Die Applikation auf die Tabletten erfolgte analog Beispiel 3.

### Beispiel 15

| Coatingzusammensetzung: | |
|---|---|
| Polyvinylalkohol-Polyethylenglykol 6000 75:25-Pfropfcopolymer (Verseifungsgrad 92 mol%) | 40 % |
| Vinylpyrrolidon-Vinylacetat 6:4 Copolymer | 30 % |
| Mikrokristalline Cellulose, mittlere | 20 % |
| Teilchengröße 20 mm | |
| Titandioxid | 5 % |
| Eisenoxid braun | 5 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 32% hergestellt. Schon nach 13 min war die Zubereitung vollständig redispergiert und gebrauchsfertig. Die Applikation auf die Tabletten erfolgte analog Beispiel 1.

### Beispiel 16

| Coatingzusammensetzung: | |
|---|---|
| Polyvinylalkohol-Polyethylenglykol 6000 (75:25)-Pfropfcopolymer (Verseifungsgrad 94 mol%) | 40 % |
| Vinylpyrrolidon-Vinylacetat 6:4 Copolymer | 15 % |
| (Copolyvidon) | |
| Mikrokristalline Cellulose, mittlere | 10 % |
| Teilchengröße 20 mm | |
| Carrageenan | 5 % |
| Talkum | 10 % |
| Calciumhydrogenphosphat | 10 % |
| Titandioxid | 5 % |
| Eisenoxid gelb | 5 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 37 % hergestellt. Schon nach 15 min war die Zubereitung vollständig redispergiert und gebrauchsfertig. Die Applikation auf die Tabletten erfolgte analog Beispiel 1.

### Beispiel 17

| Coatingzusammensetzung: | |
|---|---|
| Polyvinylalkohol-Polyethylenglykol 6000 (75:25)-Pfropfcopolymer (Verseifungsgrad 94 mol%) | 51 % |
| Vinylpyrrolidon-Vinylacetat 6:4 Copolymer | 15 % |
| (Copolyvidon) | |
| Mikrokristalline Cellulose, mittlere | 10 % |
| Teilchengröße 20 mm | |
| Polyethylenoxid-Polypropylenoxid-Blockcopolymer | 5 % |
| Poloxamer 407 | |
| Hochdisperse Kieselsäure, spezifische Oberfläche 200 m²/g | 4 % |
| Talkum | 8 % |
| Titandioxid | 5 % |
| Eisenoxid rot | 3 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 25 % hergestellt. Schon nach 8 min war die Zubereitung vollständig redispergiert und gebrauchsfertig. Die Applikation auf die Tabletten erfolgte analog Beispiel 3.

### Beispiel 18

| Coatingzusammensetzung: | |
|---|---|
| Polyvinylalkohol-Polyethylenglykol 6000 (75:25)-Pfropfcopolymer (Verseifungsgrad 94 mol%) | 30 % |
| Vinylpyrrolidon-Vinylacetat 6:4 Copolymer (Copolyvidon) | 10 % |
| Mikrokristalline Cellulose, mittlere | 10 % |
| Teilchengröße 20 mm | |
| Polyethylenoxid-Polypropylenoxid-Blockcopolymer | 4 % |
| Poloxamer 407 | |
| Hochdisperse Kieselsäure, spezifische | 4 % |
| Oberfläche 200 m^{2/g} | |
| Talkum | 40 % |
| Patentblau | 1,5% |
| Natriumlaurylsulfat | 0,5 |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 25 % hergestellt. Schon nach 7 min war die Zubereitung vollständig redispergiert und gebrauchsfertig. Die Applikation auf die Tabletten erfolgte analog Beispiel 3.

### Beispiel 19

| Coatingzusammensetzung: | |
|---|---|
| Polyvinylalkohol-Polyethylenglykol 6000 (75:25)-Pfropfcopolymer (Verseifungsgrad 94 mol%) | 30 % |
| Polyvinylalkohol (Verseifungsgrad 88 mol%, Viskosität 4 mPas) | 40 % |
| Mikrokristalline Cellulose, mittlere | 10 % |
| Teilchengröße 20 mm | |
| Polyethylenoxid-Polypropylenoxid-Blockcopolymer Poloxamer 407 | 5 % |
| Hochdisperse Kieselsäure, spezifische Oberfläche 200 m²/g | 5 % |
| Talkum | 5 % |
| Titandioxid | 5 % |

### Herstellung:

Alle Bestandteile wurden aus 25%iger Lösung sprühgetrocknet

Aus diesem Prämix wurde durch Einrühren in Wasser mittels eines Flügelrührers eine Sprühsuspension mit einem Feststoffgehalt von 25 % hergestellt. Schon nach 10 min war die Zubereitung vollständig redispergiert und gebrauchsfertig. Die Applikation auf die Tabletten erfolgte analog Beispiel 1.

Zur Applikation von farbigen Coatings können der Sprühsuspension beliebige Farbstoffe zugesetzt werden.

### Beispiel 20

| Coatingzusammensetzung: | |
|---|---|
| Polyvinylalkohol-Polyethylenglykol 6000 (75:25)-Pfropfcopolymer (Verseifungsgrad 94 mol%) | 40 % |
| Polyvinylalkohol (Verseifungsgrad 88 mol%, Viskosität 4mPas) | 30 % |
| Mikrokristalline Cellulose, mittlere Teilchengröße 20 mm | 10 % |
| Mannitol | 5 % |
| Hochdisperse Kieselsäure, spezifische Oberfläche 200 m²/g | 5 % |
| Talkum | 5 % |
| Titandioxid | 5 % |

### Herstellung:

Ein sprühgetrocknetes Pulver aus Polyvinylalkohol-Polyethylenglykol 6000 (75:25)-Pfropfcopolymer und Polyvinylalkohol (Verseifungsgrad 88 mol%, Viskosität 4 mPas) im Verhältnis 4 : 3 wurde im Turbulamischer 10 min mit den restlichen Bestandteilen gemischt.

Aus diesem Prämix wurde durch Einrühren in Wasser mittels eines Flügelrührers eine Sprühsuspension mit einem Feststoffgehalt von 25 % hergestellt. Schon nach 11 min war die Zubereitung vollständig redispergiert und gebrauchsfertig. Die Applikation auf die Tabletten erfolgte analog Beispiel 1.

Zur Applikation von farbigen Coatings können der Sprühsuspension beliebige Farbstoffe zugesetzt werden.

### Beispiel 21

| Coatingzusammensetzung: | |
|---|---|
| Polyvinylalkohol-Polyethylenglykol 6000 (75:25)-Pfropfcopolymer (Verseifungsgrad 96 mol%) | 55 % |
| Hydroxypropylmethylcellulose, Substitutionstyp 2910, Viskosität 2 gew.-%ige wässrige | 12 % |
| Lösung: 3 mPas | |
| Talkum | 20 % |
| Titandioxid | 10 % |
| Azorubinlack | 3 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 28 % hergestellt. Schon nach 12 min war die Zubereitung vollständig redispergiert und gebrauchsfertig. Die Applikation auf die Tabletten erfolgte analog Beispiel 3.

### Beispiel 22

| Coatingzusammensetzung: | |
|---|---|
| Polyvinylalkohol-Polyethylenglykol 6000 (75:25)-Pfropfcopolymer (Verseifungsgrad 94 mol%) | 50 % |
| Butylmethacrylat-2-Dimethylaminoethylmethacrylat-Methylmethacrylat (1:2:1)-Copolymer | 10 % |
| Citronensäure | 2,5 % |
| Mikrokristalline Cellulose, mittlere Teilchengröße 20 mm | 20 % |
| Natriumlaurylsulfat | 0,5 % |
| Titandioxid | 5 % |
| Talkum | 7 % |
| Eisenoxid braun | 5 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 28 % hergestellt. Schon nach 11 min war die Zubereitung vollständig redispergiert und gebrauchsfertig. Die Applikation auf die Tabletten erfolgte analog Beispiel 1.

### Beispiel 23

| Coatingzusammensetzung: | |
|---|---|
| Polyvinylalkohol-Polyethylenglykol 6000 (75:25)-Pfropfcopolymer (Verseifungsgrad 96 mol%) | 50 % |
| Hydroxypropylcellulose, Substitutionsgrad 3,4-4,1, Viskosität 10 gew.-%ige wässrige Lösung 300-600 mPas | 15 % |
| Talkum | 22 % |
| Titandioxid | 10 % |
| Chinolingelblack | 3 % |

### Herstellung:

Alle Bestandteile wurden in einem Turbulamischer 10 min gemischt.

Aus diesem Prämix wurde durch Einrühren in Wasser mittels eines Flügelrührers eine Sprühsuspension mit einem Feststoffgehalt von 28 % hergestellt. Schon nach 12 min war die Zubereitung vollständig redispergiert und gebrauchsfertig. Die Applikation auf die Tabletten erfolgte analog Beispiel 3.

## Patentansprüche

1. In Wasser schnelllösliches Filmüberzugsmittel zum Überziehen von pharmazeutischen, kosmetischen oder agrochemische Darreichungsformen, Saatgut, Nahrungsergänzungsmittel oder Lebensmitteln als festen Substraten, bestehend aus
a) 10-90 Gew.-% eines Polyvinylalkohol-Polyether-Pfropfcopolymeren (Komponente A),
b) 5-80 Gew.-% mindestens einer weiteren Komponente, die mindestens eine funktionelle Gruppe ausgewählt aus der Gruppe bestehend aus Hydroxy-, Amid- und Esterfunktionen enthält und ein Polymer ausgewählt aus der Gruppe bestehend aus Polysacchariden, Cellulosen, Stärken, Gelatine, Polyvinylpyrrolidonen, Vinylpyrrolidon-Vinylacetat-Copolymeren, Vinylpyrrolidon-Methacrylat-Copolymeren, Vinylpyrrolidon-Acrylat-Copolymeren, (Meth)-acrylat-Copolymeren, Hydroxyalkyl(meth)acrylat-Copolymeren, Polyvinylacetaten, Polylactiden, Polyethylenglykolen, Polypropylenglykolen oder Polyethylenglykol-Polypropylen-glykol-Blockcopolymeren und deren Derivaten, oder ein Zucker- oder Zuckeralkohol oder eine Derivat davon, Harnstoff oder eine hochdisperse Kieselsäure mit einer spezifischen Oberfläche ≥ 100 m²/g (Komponente B) darstellt, sowie
c) 0-70 Gew.-% weiteren üblichen Coatingbestandteilen (Komponenten C).

2. Filmüberzugsmittel nach Anspruch 1, **dadurch gekennzeichnet, dass** Komponente B eine Verbindung ausgewählt aus der Gruppe bestehend aus Lactose, Saccharose, Glucose, Xylose, Mannit, Sorbit, Xylit und Isomalt ist.

3. Filmüberzugsmittel nach einem der Ansprüche 1 oder 2, **dadurch gekennzeichnet, dass** Komponente B ausgewählt ist aus der Gruppe bestehend aus Hydroxypropylmethylcellulose, Hydroxypropylcellulose, Hydroxyethylcellulose, Hydroxyethylmethylcellulose, Methylcellulose, Ethylcellulose, Carboxymethylcellulose, Cellulose und mikrokristalline Cellulose.

4. Filmüberzugsmittel nach einem der Ansprüche 1 bis3, **dadurch gekennzeichnet, dass** Komponente B ausgewählt ist aus der Gruppe bestehend aus Alginaten, Propylenglykolalginaten, Carrageenanen, Pectinen, Guar, Tara, Xanthanen, Gummi arabicum oder Chitosanen und deren Salzen.

5. Filmüberzugsmittel nach einem der Ansprüche 1 bis4, **dadurch gekennzeichnet, dass** Komponente B ausgewählt ist aus der Gruppe bestehend aus N-Vinylpyrrolidon-Homopolymeren, quervernetzte Polyvinylpyrrolidone, Polyvinylacetat und N-Vinylpyrrolidon-Vinylacetat-Copolymeren.

6. Filmüberzugsmittel nach einem der Ansprüche 1 bis 5 , **dadurch gekennzeichnet, dass** als Komponente B Vinylpyrrolidon-Acrylat-Copolymere oder Vinylpyrrolidon-Methacrylat-Copolymere eingesetzt werden.

7. Filmüberzugsmittel nach einem der Ansprüche 1 bis6, **dadurch gekennzeichnet, dass** als Komponente B (Meth)acrylat-Copolymere oder Hydroxyalkyl(meth)acrylat-Copolymere eingesetzt werden.

8. Filmüberzugsmittel nach einem der Ansprüche 1 bis7, **dadurch gekennzeichnet, dass** als Komponente B ein Butylmethacrylat-2-Dimethylaminoethylmeth-crylat-Methylmethacrylat (1:2:1)-Copolymer, ein Methacrylsäure-Methylmeth-crylat-Copolymer oder ein Methacrylsäure-Ethylacrylat (1:1)-Copolymer oder deren Salze eingesetzt werden.

9. Filmüberzugsmittel nach einem der Ansprüche 1 bis8, **dadurch gekennzeichnet, dass** als Komponente B Stärke, Stärkederivate, Stärkehydrolisate, Amylose, Cyclodextrine, Maltodextrine, Glucosesirupe, Dextrane, Inulin, Polydextrose oder Polyfructose eingesetzt werden.

10. Filmüberzugsmittel nach einem der Ansprüche 1 bis9, **dadurch gekennzeichnet**, als Komponente B Lactose, Glucose, Xylose oder Saccharose eingesetzt werden.

11. Filmüberzugsmittel nach einem der Ansprüche 1 bis 10, **dadurch gekennzeichnet, dass** als Komponente B Isomalt eingesetzt wird.

12. Filmüberzugsmittel nach einem der Ansprüche 1 bis 11, **dadurch gekennzeichnet, dass** als Komponente B Mannitol, Sorbitol oder Xylitol eingesetzt werden.

13. Filmüberzugsmittel nach Anspruch 1, **dadurch gekennzeichnet, dass** als Komponente B Harnstoff eingesetzt wird.

14. Filmüberzugsmittel nach einem der Ansprüche 1 bis 14, **dadurch gekennzeichnet, dass** als Komponente B eine hochdisperse Kieselsäure mit einer spezifischen Oberfläche > 100 m²/g eingesetzt wird.

15. Filmüberzugsmittel nach einem der Ansprüche 1 bis 14, **dadurch gekennzeichnet, dass** als Komponenten C Farbstoffe, Farblacke, Pigmente, Antiklebemittel, Füllstoffe, Glanzverstärker, Benetzungsmittel, Tenside, Schaumverhinderer, Schutzkolloide, Puffersubstanzen, pH-regulierenden Substanzen oder Weichmacher eingesetzt werden.

16. Verfahren zur Herstellung überzogener Substrate, **dadurch gekennzeichnet, dass** ein Filmüberzugsmittel gemäß einem der Ansprüche 1 bis 15 in Wasser eingerührt und mittels einer geeigneten Sprüheinrichtung auf das Substrat aufgebracht wird, wobei durch Zufuhr von erwärmter Luft der Filmüberzug sukzessive getrocknet wird.

17. Verfahren nach Anspruch16, **dadurch gekennzeichnet, dass** das Filmüberzugsmittel vor der Zugabe zu Wasser entweder trocken gemischt oder kompaktiert oder granuliert wird und als Vormischung in Wasser gegeben wird.

18. Verfahren nach Anspruch16, **dadurch gekennzeichnet, dass** die Komponenten A und B sowie die Komponenten C mit Ausnahme der farbgebenden Komponenten trocken gemischt oder kompaktiert oder granuliert werden und diese Zubereitung vor oder gemeinsam mit den farbgebenden Komponenten in Wasser eingerührt wird.

19. Verfahren nach einem Ansprüche 16 bis18, **dadurch gekennzeichnet, dass** eine Mischung der Komponenten A und B aus wässriger Lösung sprühgetrocknet, wirbelschichtgetrocknet oder walzengetrocknet wird und dieses Pulver mit den Komponenten C in Wasser eingerührt wird.

20. Verfahren nach einem der Ansprüche 16 bis19, **dadurch gekennzeichnet, dass** eine Mischung der Komponenten A und B aus wässriger Lösung sprühgetrocknet, wirbelschichtgetrocknet oder walzengetrocknet werden und dieses Pulver mit den Komponenten C gemischt oder kompaktiert oder granuliert wird und als solche Mischung in Wasser eingerührt wird.

21. Verfahren nach einem der Ansprüche 16 bis 20, **dadurch gekennzeichnet, dass** die Komponenten A und B sowie die Komponenten C mit Ausnahme der farbgebenden Komponenten aus wässriger Lösung sprühgetrocknet, wirbelschichtgetrocknet oder walzengetrocknet werden und das resultierende Pulver gegebenenfalls mit der farbgebenden Komponente in Wasser eingerührt wird.

22. Feste Substrate, wobei als Substrate pharmazeutische, kosmetische, agrochemische Darreichungsformen, Saatgut, Nahrungsergänzungsmittel oder Lebensmittel eingesetzt werden, überzogen mit einem Filmüberzug gemäß einem der Ansprüche 1 bis15.
